# EUROPEAN PATENT APPLICATION

(11) **EP 4 253 386 A1**
(43) Date of publication of application: **04.10.2023**
(21) Application number: 21897018.4
(22) Date of filing: 24.11.2021
(51) Int. Cl.: C07D 495/04, A61K 31/4365, A61P 35/00

(54) **DEUTERIUM-MODIFIED THIENOPYRIDONE COMPOUND**

(30) Priority: 24.11.2020 CN 202011334883
(71) Applicant: CHIA TAI TIANQING PHARMACEUTICAL GROUP CO., LTD., Lianyungang, Jiangsu 222062 (CN)
(72) Inventor: ZHANG, Yinsheng, Nanjing, Jiangsu 211100 (CN); GAO, Yong, Nanjing, Jiangsu 211100 (CN); YIN, Yuan, Nanjing, Jiangsu 211100 (CN); SHI, Wei, Nanjing, Jiangsu 211100 (CN); ZHAO, Damin, Nanjing, Jiangsu 211100 (CN); ZHU, Gaoyuan, Nanjing, Jiangsu 211100 (CN); WANG, Xiaojin, Nanjing, Jiangsu 211100 (CN); JI, Yanpeng, Nanjing, Jiangsu 211100 (CN)
(74) Representative: Hoffmann Eitle
(86) International application number: PCT/CN2021/132748
(87) International publication number: WO 2022/111517

(57) **Abstract**

The present disclosure provides a deuterium-modified thienopyridone compound, a preparation method therefor, a pharmaceutical composition thereof, and a medical use thereof. The deuterium-modified thienopyridone compound is shown in formula I, and has been shown to inhibit HPK1 kinase activity, being capable of achieving an anti-tumour effect.

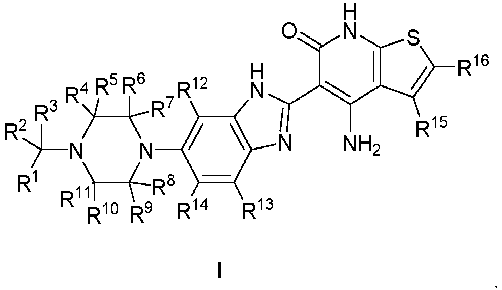

## Description

### TECHNICAL FIELD

The present application is in the field of pharmaceutical chemistry and relates to a deuterium-modified thienopyridone compound and, in particular, to a compound of formula **I** or a pharmaceutically acceptable salt thereof, a preparation method therefor, a pharmaceutical composition thereof and medical use thereof. The deuterium-modified thienopyridone compound of the present application is shown to inhibit the HPK1 kinase activity and can achieve an anti-tumor effect.

### BACKGROUND

Hematopoietic progenitor kinase 1 (HPK1), also known as mitogen-activated protein kinase 1 (MAP4K1), is a mammalian Ste20-associated serine/threonine protein kinase. It is a microtubule-associated protein and also a member of the mitogen-activated protein kinase (MAP4K) family. The family also includes five subtypes: GCK/MAP4K2, GLK/MAP4K3, HGK/MAP4K4, KHS1/MAP4K5 and MINK1/MAP4K6. Unlike the other five MAP4K subtypes that are extensively expressed in histiocytes, HPK1 is expressed only in hematopoietic tissue cells and can participate in the regulation of signal transduction in the hematopoietic system, including lymphocytes, by mediating a variety of cellular signaling pathways, including MAPK signaling, antigen receptor signaling, cytokine signaling, and the like.

Research shows that HPK1 functions mainly through the c-Jun N-terminal kinase (JNK) and extracellular regulated protein kinases (ERK) signaling pathways, inhibiting immune cell responses. In T cells, after T cell receptor (TCR) proteins are activated, HPK1 interacts with a large number of TCRs and is phosphorylated by tyrosine kinases Lck and Zap70, and the activated HPK1 further phosphorylates T cell receptor adaptor protein SLP-76 to establish a docking site for negative regulatory factor 14-3-3 and finally undermines the stability of the TCR signaling complex (lato-gads-SLP76) and blocks the transmission of downstream mitogen-activated protein (MAP) kinase signals, negatively regulating TCR signal transduction and further inhibiting T cell proliferation. In B cells, a similar negative feedback mechanism also exists. B cell receptor (BCR) signals are transmitted through HPK1-mediated phosphorylation and activated B cell linker protein (BLNK), so that downstream signal transmission is blocked and B cell proliferation is inhibited. In addition, HPK1 also has a negative feedback regulatory effect on NK (natural killer) cells and dendritic cells (DCs).

Being important for immunity, HPK1 inhibitors play an important role in malignant solid tumors or hematological cancers (e.g., acute myeloid leukemia, breast cancer and lung cancer), autoimmune diseases (e.g., systemic lupus erythematosus and psoriatic arthritis) and inflammatory responses.

There have now been no HPK1-targeted drugs available on the market. There remains a need in the art to develop compounds with selective inhibitory activity, better pharmacodynamics, or better pharmacokinetics.

### BRIEF SUMMARY

The present application provides a compound of formula (I) or a pharmaceutically acceptable salt thereof, 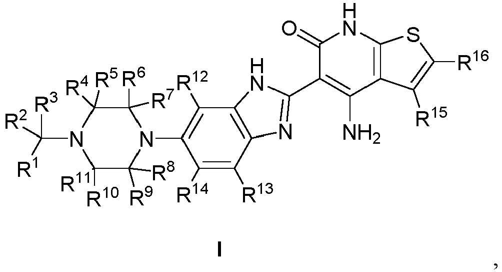 wherein,
R¹, R², R³, R⁴, R⁵, R⁶, R⁷, R⁸, R⁹, R¹⁰, R¹¹, R¹², R¹³, R¹⁴, R¹⁵ and R¹⁶ are each independently selected from the group consisting of hydrogen (H) and deuterium (D), provided that at least one of R¹, R², R³, R⁴, R⁵, R⁶, R⁷, R⁸, R⁹, R¹⁰, R¹¹, R¹², R¹³, R¹⁴, R¹⁵ and R¹⁶ is selected from deuterium.

In some embodiments, at least two, three, four, five, six, seven, eight, nine, ten, eleven, twelve, thirteen, fourteen, fifteen or sixteen of the R¹, R², R³, R⁴, R⁵, R⁶, R⁷, R⁸, R⁹, R¹⁰, R¹¹, R¹², R¹³, R¹⁴, R¹⁵ and R¹⁶ are selected from deuterium.

In some embodiments, any one, two, three, four, five, six, seven, eight, nine, ten, eleven, twelve, thirteen, fourteen, fifteen or sixteen of the R¹, R², R³, R⁴, R⁵, R⁶, R⁷, R⁸, R⁹, R¹⁰, R¹¹, R¹², R¹³, R¹⁴, R¹⁵ and R¹⁶ are selected from deuterium.

In some embodiments, the R¹, R² and R³ are selected from deuterium, and R⁴, R⁵, R⁶, R⁷, R⁸, R⁹, R¹⁰, R¹¹, R¹², R¹³, R¹⁴, R¹⁵ and R¹⁶ are each independently selected from the group consisting of hydrogen and deuterium.

In some embodiments, the R⁴, R⁵, R¹⁰ and R¹¹ are selected from deuterium, and R¹, R², R³, R⁶, R⁷, R⁸, R⁹, R¹², R¹³, R¹⁴, R¹⁵ and R¹⁶ are each independently selected from the group consisting of hydrogen and deuterium.

In some embodiments, the R⁶, R⁷, R⁸ and R⁹ are selected from deuterium, and R¹, R², R³, R⁴, R⁵, R¹⁰, R¹¹, R¹², R¹³, R¹⁴, R¹⁵ and R¹⁶ are each independently selected from the group consisting of hydrogen and deuterium.

In some embodiments, the R¹, R², R³, R⁴, R⁵, R¹⁰ and R¹¹ are selected from deuterium, and R⁶, R⁷, R⁸, R⁹, R¹², R¹³, R¹⁴, R¹⁵ and R¹⁶ are each independently selected from the group consisting of hydrogen and deuterium.

In some embodiments, the R¹, R², R³, R⁶, R⁷, R⁸ and R⁹ are selected from deuterium, and R⁴, R⁵, R¹⁰, R¹¹, R¹², R¹³, R¹⁴, R¹⁵ and R¹⁶ are each independently selected from the group consisting of hydrogen and deuterium.

In some embodiments, the R⁴, R⁵, R⁶, R⁷, R⁸, R⁹, R¹⁰ and R¹¹ are selected from deuterium, and R¹, R², R³, R¹², R¹³, R¹⁴, R¹⁵ and R¹⁶ are each independently selected from the group consisting of hydrogen and deuterium.

In some embodiments, the R¹, R², R³, R⁴, R⁵, R⁶, R⁷, R⁸, R⁹, R¹⁰ and R¹¹ are selected from deuterium, and R¹², R¹³, R¹⁴, R¹⁵ and R¹⁶ are each independently selected from the group consisting of hydrogen and deuterium.

In some embodiments, the R¹² and R¹⁴ are selected from deuterium, and R¹, R², R³, R⁴, R⁵, R⁶, R⁷, R⁸, R⁹, R¹⁰, R¹¹, R¹³, R¹⁵ and R¹⁶ are each independently selected from the group consisting of hydrogen and deuterium.

In some embodiments, the R¹³ is selected from deuterium, and R¹, R², R³, R⁴, R⁵, R⁶, R⁷, R⁸, R⁹, R¹⁰, R¹¹, R¹², R¹⁴, R¹⁵ and R¹⁶ are each independently selected from the group consisting of hydrogen and deuterium.

In some embodiments, the R¹², R¹³ and R¹⁴ are selected from deuterium, and R¹, R², R³, R⁴, R⁵, R⁶, R⁷, R⁸, R⁹, R¹⁰, R¹¹, R¹⁵ and R¹⁶ are each independently selected from the group consisting of hydrogen and deuterium.

In some embodiments, the R¹, R², R³, R¹², R¹³ and R¹⁴ are selected from deuterium, and R⁴, R⁵, R⁶, R⁷, R⁸, R⁹, R¹⁰, R¹¹, R¹⁵ and R¹⁶ are each independently selected from the group consisting of hydrogen and deuterium.

In some embodiments, the R¹⁵ is selected from deuterium, and R¹, R², R³, R⁴, R⁵, R⁶, R⁷, R⁸, R⁹, R¹⁰, R¹¹, R¹², R¹³, R¹⁴ and R¹⁶ are each independently selected from the group consisting of hydrogen and deuterium.

In some embodiments, the R¹⁶ is selected from deuterium, and R¹, R², R³, R⁴, R⁵, R⁶, R⁷, R⁸, R⁹, R¹⁰, R¹¹, R¹², R¹³, R¹⁴ and R¹⁵ are each independently selected from the group consisting of hydrogen and deuterium.

In some embodiments, the R¹⁵ and R¹⁶ are selected from deuterium, and R¹, R², R³, R⁴, R⁵, R⁶, R⁷, R⁸, R⁹, R¹⁰, R¹¹, R¹², R¹³ and R¹⁴ are each independently selected from the group consisting of hydrogen and deuterium.

In some embodiments, the R¹, R², R³, R¹⁵ and R¹⁶ are selected from deuterium, and R⁴, R⁵, R⁶, R⁷, R⁸, R⁹, R¹⁰, R¹¹, R¹², R¹³ and R¹⁴ are each independently selected from the group consisting of hydrogen and deuterium.

In some embodiments, the compound of formula I or the pharmaceutically acceptable salt thereof is selected from the group consisting of the following compounds or pharmaceutically acceptable salts thereof:

| Compound | R¹ | R² | R³ | R⁴ | R⁵ | R⁶ | R⁷ | R⁸ | R⁹ | R¹⁰ | R¹¹ | R¹² | R¹³ | R¹⁴ | R¹⁵ | R¹⁶ |
|---|---|---|---|---|---|---|---|---|---|---|---|---|---|---|---|---|
| I-1 | D | D | D | H | H | H | H | H | H | H | H | H | H | H | H | H |
| I-2 | H | H | H | D | D | D | D | D | D | D | D | H | H | H | H | H |
| I-3 | H | H | H | D | D | H | H | H | H | D | D | H | H | H | H | H |
| I-4 | H | H | H | H | H | D | D | D | D | H | H | H | H | H | H | H |
| I-5 | D | D | D | D | D | D | D | D | D | D | D | H | H | H | H | H |
| I-6 | D | D | D | D | D | H | H | H | H | D | D | H | H | H | H | H |
| I-7 | H | H | H | H | H | H | H | H | H | H | H | D | D | D | H | H |
| I-8 | H | H | H | H | H | H | H | H | H | H | H | H | H | H | D | D |
| I-9 | D | D | D | H | H | H | H | H | H | H | H | D | D | D | H | H |
| I-10 | D | D | D | H | H | H | H | H | H | H | H | H | H | H | D | D |
| I-11 | H | H | H | H | H | H | H | H | H | H | H | D | H | D | H | D |

In some embodiments, the compound of formula I or the pharmaceutically acceptable salt thereof is selected from the group consisting of the following compounds or pharmaceutically acceptable salts thereof:

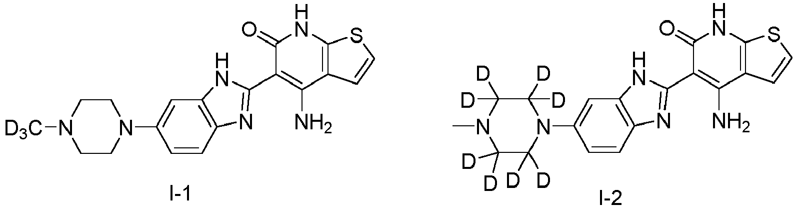

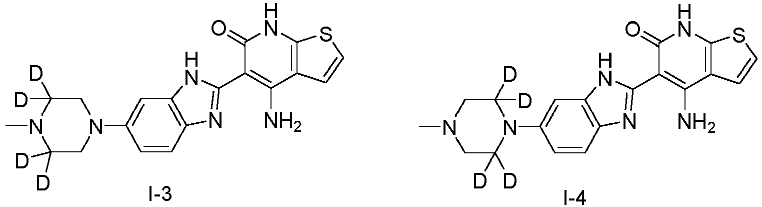

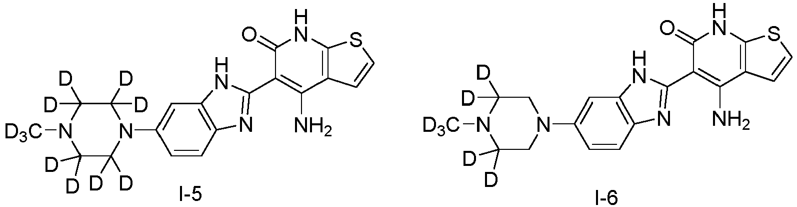

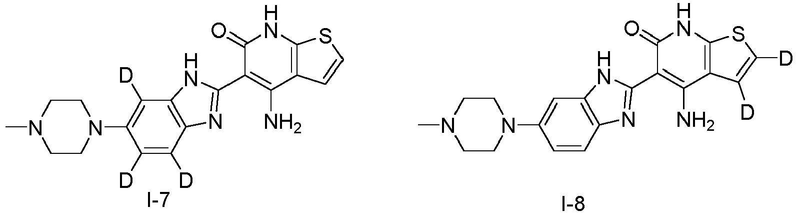

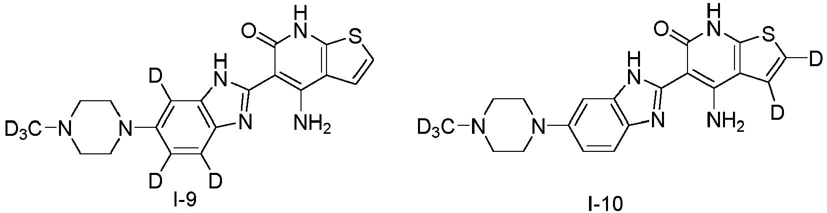

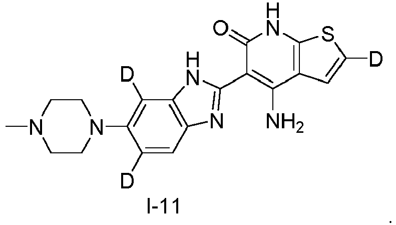

Another aspect of the present application provides a pharmaceutical composition comprising the compound or the pharmaceutically acceptable salt thereof of the present application and optionally further comprising a pharmaceutically acceptable excipient. In one embodiment, the pharmaceutical composition further comprises a second active agent, wherein the second active agent is other anti-cancer agent (e.g., a small-molecule anti-cancer agent or an antibody anti-cancer agent).

Another aspect of the present application provides use of the compound or the pharmaceutically acceptable salt thereof of the present application or the pharmaceutical composition described above for preparing a medicament for treating a disease that benefits from inhibiting the HPK1 kinase activity. In one embodiment, the medicament further comprises a second active agent for treating the disease, wherein the second active agent is other anti-cancer agent (e.g., a small-molecule anti-cancer agent or an antibody anti-cancer agent).

Another aspect of the present application provides use of the compound or the pharmaceutically acceptable salt thereof of the present application or the pharmaceutical composition described above in treating a disease that benefits from inhibiting the HPK1 kinase activity.

Another aspect of the present application provides the compound or the pharmaceutically acceptable salt thereof of the present application or the pharmaceutical composition described above for use in treating a disease that benefits from inhibiting the HPK1 kinase activity.

Another aspect of the present application provides a method for treating a disease that benefits from inhibiting the HPK1 kinase activity comprising administering to an individual in need thereof (the individual is selected from a mammal, preferably a human) a therapeutically effective amount of the compound or the pharmaceutically acceptable salt thereof of the present application or the pharmaceutical composition described above.

In some embodiments, the disease that benefits from inhibiting the HPK1 kinase activity is selected from the group consisting of tumors and cancer, e.g., hematological cancer and solid tumors, e.g., acute myeloid leukemia, breast cancer and lung cancer.

In one embodiment, in the method for treating the disease described above, the compound or the pharmaceutically acceptable salt thereof of the present application or the pharmaceutical composition described above is administered in conjunction with other anti-cancer agent (e.g., a small-molecule anti-cancer agent or an antibody anti-cancer agent) or other anti-cancer therapy (e.g., radiotherapy and/or chemotherapy).

The compound of the present application has good HPK1 *in vitro* kinase inhibitory activity and cellular p-SLP76 phosphorylation inhibitory activity, as well as good *in vitro* liver microsomal stability.

### DETAILED DESCRIPTION

### Definitions

Unless otherwise stated, the following terms used in the present application shall have the following meanings. A certain term, unless otherwise specifically defined, should not be considered uncertain or unclear, but construed according to its common meaning in the field. When referring to a trade name herein, it is intended to refer to its corresponding commercial product or its active ingredient.

The word "comprise" and variations thereof such as "comprises" or "comprising" will be understood in an open, non-exclusive sense, i.e., "including but not limited to".

The term "independently" means that when any variable (e.g., R) occurs more than once in the composition or structure of a compound, the variable is independently defined in each case. For example, if a group is substituted with 2 R, the definition of each R is independent. Or, for example, if multiple groups are substituted with an R, the cases where each group is substituted with an R are independent of each other.

The term "pharmaceutically acceptable" is used herein for those compounds, materials, compositions, and/or dosage forms which are, within the scope of sound medical judgment, suitable for use in contact with the tissues of human beings and animals without excessive toxicity, irritation, allergic response, or other problems or complications, and commensurate with a reasonable benefit/risk ratio.

A pharmaceutically acceptable salt, for example, may be a metal salt, an ammonium salt, a salt formed with an organic base, a salt formed with an inorganic acid, a salt formed with an organic acid, a salt formed with a basic or acidic amino acid, and the like.

The term "pharmaceutical composition" refers to a mixture consisting of one or more of the compounds or the salts thereof of the present application and a pharmaceutically acceptable excipient. The pharmaceutical composition is intended to facilitate the administration of the compound of the present application to an organic entity.

The term "pharmaceutically acceptable excipients" refers to those which do not have a significant irritating effect on an organic entity and do not impair the biological activity and properties of the active compound. Suitable excipients are well known to those skilled in the art, for example, carbohydrate, wax, water-soluble and/or water-swellable polymers, hydrophilic or hydrophobic material, gelatin, oil, solvent, water, and the like.

The pharmaceutical composition of the present application can be prepared by combining the compound of the present application with a suitable pharmaceutically acceptable excipient, and can be formulated, for example, into a solid, semisolid, liquid, or gaseous formulation, such as tablet, pill, capsule, powder, granule, ointment, emulsion, suspension, suppository, injection, inhalant, gel, microsphere and aerosol.

Typical routes of administration of the compound or the pharmaceutically acceptable salt thereof or the pharmaceutical composition thereof of the present application include, but are not limited to, oral, rectal, local, inhalation, parenteral, sublingual, intravaginal, intranasal, intraocular, intraperitoneal, intramuscular, subcutaneous and intravenous administration.

The term "treat", "treating" or "treatment" usually refers to acquiring needed pharmacological effect and/or physiological effect. The effect partially or fully stabilizes or cures the disease and/or a side effect of the disease, and can be therapeutic. As used herein, "treat", "treating" or "treatment" encompasses any treatment of a disease in a patient, including (a) inhibiting a symptom of the disease, i.e., blocking the progression of the disease; or (b) alleviating a symptom of the disease, i.e., causing remission of the disease or the symptom.

The term "effective amount" refers to an amount of the compound of the present application for (i) treating a specific disease, condition or disorder; (ii) alleviating, relieving or eliminating one or more symptoms of a specific disease, condition or disorder, or (iii) delaying onset of one or more symptoms of a specific disease, condition or disorder described herein. The amount of active substance (e.g., the antibody or compound of the present application) constituting the "therapeutically effective amount" may vary according to factors such as the disease state, age, sex, and weight of the individual, and the ability of a therapeutic agent or a combination of therapeutic agents to elicit a desired response in the individual. The effective amount may also be determined routinely by those skilled in the art in accordance with their knowledge and the present disclosure.

In the present application, the term "individual" includes humans and animals, e.g., mammals (e.g., primates, cows, horses, pigs, dogs, cats, mice, rats, rabbits, goats, sheep, and birds). In some embodiments, the subject or patient is a mouse. In some embodiments, the subject or patient is a human.

Therapeutic dosages of the compounds of the present application may be determined by, for example, the specific use of a treatment, the route of administration of the compound, the health and condition of a patient, and the judgment of a prescribing physician. The proportion or concentration of the compound of the present application in a pharmaceutical composition may not be constant and depends on a variety of factors including dosages, chemical properties (e.g., hydrophobicity), and routes of administration. For example, the compound of the present application may be provided for parenteral administration by a physiological buffered aqueous solution containing about 0.1-10%w/v of the compound. Certain typical dosages range from about 1 µg/kg body weight/day to about 1 g/kg body weight/day. In certain embodiments, the dosage ranges from about 0.01 mg/kg body weight/day to about 100 mg/kg body weight/day. The dosage is likely to depend on such variables as the type and degree of progression of the disease or disorder, the general health condition of the particular patient, the relative biological potency of the compound selected, the excipient formulation and its route of administration. Effective doses can be extrapolated from dose-response curves derived from *in vitro* or animal model test systems. The compounds of the present application can be prepared using a variety of synthetic methods well known to those skilled in the art, including the specific embodiments listed below, embodiments formed by combinations thereof with other chemical synthetic methods, and equivalents thereof known to those skilled in the art. The preferred embodiments include, but are not limited to, the examples of the present application.

The chemical reactions of the embodiments of the present application are carried out in a proper solvent that must be suitable for the chemical changes in the present application and the reagents and materials required. In order to acquire the compounds of the present application, it is sometimes necessary for those skilled in the art to modify or select a synthesis procedure or a reaction scheme based on the existing embodiments.

### Examples

For clarity, the present application is further described with the following examples, which are, however, not intended to limit the scope of the invention. All reagents used in the present application are commercially available and can be used without further purification.

In addition to the following examples, other compounds disclosed by the present application can be prepared by referring to the preparation methods of the examples or preparation methods known in the art. Mass spectrometry (MS) or nuclear magnetic resonance (NMR) analysis of a compound can be performed by one skilled in the art using the methods of the present application or preparation methods known in the art.

### Example 1: Preparation of

### 4-amino-5-(5-(4-(methyl-d₃)piperazin-1-yl)-1H-benzo[d]imidazol-2-yl)thieno[2,3-b]pyridi n-6(7H)-one (compound I-1)

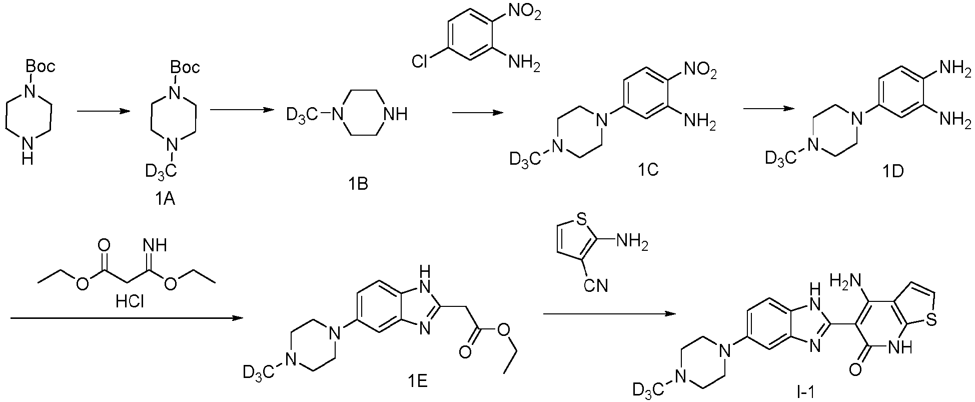

### Step 1: preparation of tert-butyl 4-(methyl-d₃)piperazine-1-carboxylate (compound 1A)

In a 250 mL reaction flask, tert-butyl piperazine-1-carboxylate (5 g) and potassium carbonate (7.42 g) were dissolved in tetrahydrofuran (100 mL). The reaction was stirred at 25 °C under nitrogen atmosphere, and iodomethane-*d*₃(4.28 g) was then added dropwise at -30 °C. The reaction was stirred at 25 °C under nitrogen atmosphere for another period of time. After the reaction was completed, the reaction mixture was filtered and concentrated under reduced pressure to give compound 1A(5.5 g).

MS: m/z = 204.2 [M+H]⁺.

### Step 2: preparation of 1-(methyl-d₃)piperazine (compound 1B)

Compound 1A (5.5 g), trifluoroacetic acid (12 g), and dichloromethane (30 mL) were added successively to a 100 mL reaction flask, and the reaction was stirred at 25 °C. After the reaction was completed, the reaction mixture was adjusted to alkalinity and extracted with dichloromethane (50 mL × 3), and the organic phases were combined, dried, filtered, and concentrated under reduced pressure to give compound 1B (2.8 g).

MS: m/z = 104.2 [M+H]⁺.

### Step 3: preparation of 5-(4-(methyl-d₃)piperazin-1-yl)-2-nitroaniline (compound 1C)

Compound 1B (2.8 g), 5-chloro-2-nitroaniline (2.7 g), potassium carbonate (8.6 g), and N-methylpyrrolidone (20 mL) were added successively to a 100 mL reaction flask, and the reaction was stirred at 160 °C. After the reaction was completed, the reaction mixture was concentrated under reduced pressure. The residue was separated by column chromatography (dichloromethane/methanol = 9/1) to give compound 1C (1.12 g).

MS: m/z = 240.2 [M+H]⁺.

### Step 4: preparation of 4-(4-(methyl-d₃)piperazin-1-yl)benzene-1,2-diamine (compound 1D)

1C (1.12 g), ethanol (20 mL), water (10 mL), zinc powder (1.6 g), and ammonium chloride (0.131 g) were added successively to a 100 mL reaction flask. The reaction was stirred at 80 °C. After the reaction was completed, the reaction mixture was filtered under vacuum, and the filtrate was concentrated under reduced pressure to give compound 1D (1 g).

MS: m/z = 210.3 [M+H]⁺.

### Step 5: preparation of ethyl 2-(5-(4-(methyl-d₃)piperazin-1-yl)-1H-benzo[d]imidazol-2-yl)acetate (compound 1E)

Ethyl 3-ethoxy-3-iminopropionate hydrochloride (1.4 g), compound 1D (1 g), and dimethylformamide (20 mL) were added successively to a 100 mL reaction flask, and the reaction was stirred at 80 °C under nitrogen atmosphere. After the reaction was completed, the reaction mixture was concentrated under reduced pressure. The residue was separated by column chromatography (dichloromethane/methanol = 4/1) to give compound 1E (1.416 g).

MS: m/z = 306.1 [M+H]⁺.

### Step 6: preparation of 4-amino-5-(5-(4-(methyl-d₃)piperazin-1-yl)-1H-benzo[d]imidazol-2-yl)thieno[2,3-b]pyridi n-6(7H)-one (compound I-1)

Compound 1E (1.416 g), 2-aminothiophene-3-carbonitrile (0.576 g), tetrahydrofuran (20 mL), and lithium diisopropylamide (4.9 g) were added successively to a 100 mL reaction flask, and the reaction was stirred at 60 °C under nitrogen atmosphere. After the reaction was completed, the reaction mixture was quenched with saturated ammonium chloride and extracted with ethyl acetate (30 mL × 3), and the organic phases were combined, dried, filtered, and concentrated under reduced pressure. The residue was separated by column chromatography (dichloromethane/methanol = 9/1) to give compound 1-1 (0.1 g).

MS: m/z = 384.6 [M+H]⁺.

¹H NMR (500 MHz, DMSO-*d*₆) δ 12.62(s, 1H), 12.10(s, 1H), 10.67(d, 1H), 8.00(s, 1H), 7.45-7.60(m, 2H), 7.19(s, 2H), 6.89(s, 1H), 3.13(s, 4H), 2.60(s, 4H).

### Example 2: Preparation of

### 4-amino-5-(5-(4-methylpiperazin-1-yl-2,2,3,3,5,5,6,6-d₈)-1H-benzo[d]imidazol-2-yl)thieno [2,3-b]pyridin-6(7H)-one (compound I-2)

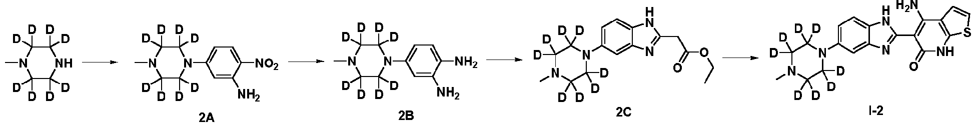

### Step 1: preparation of 5-(4-methylpiperazin-1-yl-2,2,3,3,5,5,6,6-d₈)-2-nitroaniline (compound 2A)

Compound 1-methylpiperazine-2,2,3,3,5,5,6,6-*d*₈ (0.847 g), 5-chloro-2-nitroaniline (1.356 g), potassium carbonate (4.330 g), and N-methylpyrrolidone (20 mL) were added successively to a 100 mL reaction flask, and the reaction was stirred at 160 °C. After the reaction was completed, the reaction mixture was concentrated under reduced pressure. The residue was separated by column chromatography (dichloromethane/methanol = 9/1) to give compound 2A(0.619 g).

MS: m/z = 245.1 [M+H]⁺.

### Step 2: preparation of 4-(4-methylpiperazin-1-yl-2,2,3,3,5,5,6,6-d₈)benzene-1,2-diamine (compound 2B)

Compound 2A (0.618 g), ethanol (30 mL), water (5 mL), zinc powder (0.828 g), and ammonium chloride (1.354 g) were added successively to a 100 mL reaction flask. The reaction was stirred at 80 °C. After the reaction was completed, the reaction mixture was filtered under vacuum, and the filtrate was concentrated under reduced pressure to give compound 2B (0.543 g).

MS: m/z = 215.2 [M+H]⁺.

### Step 3: preparation of ethyl 2-(5-(4-methylpiperazin-1-yl-2,2,3,3,5,5,6,6-d₈)-1H-benzo[d]imidazol-2-yl)acetate (compound 2C)

Ethyl 3-ethoxy-3-iminopropionate hydrochloride (0.826 g), compound 2B (0.543 g), and dimethylformamide (10 mL) were added successively to a 100 mL reaction flask, and the reaction was stirred at 80 °C under nitrogen atmosphere. After the reaction was completed, the reaction mixture was concentrated under reduced pressure. The residue was separated by column chromatography (dichloromethane/methanol = 4/1) to give compound 2C (0.314 g).

MS: m/z = 311.5 [M+H]⁺.

### Step 4: preparation of 4-amino-5-(5-(4-methylpiperazin-l-yl-2,2,3,3,5,5,6,6-d₈)-1H-benzo[d]imidazol-2-yl)thieno [2,3-b]pyridin-6(7H)-one (compound I-2)

Compound 2C (0.310 g), 2-aminothiophene-3-carbonitrile (0.200 g), tetrahydrofuran (30 mL), and a 2 M solution of lithium diisopropylamide in tetrahydrofuran (5 mL) were added successively to a 100 mL reaction flask, and the reaction was stirred at 60 °C under nitrogen atmosphere. After the reaction was completed, the reaction mixture was quenched with saturated ammonium chloride and extracted with ethyl acetate, and the organic phases were combined, dried, filtered, and concentrated under reduced pressure. The residue was separated by column chromatography (dichloromethane/methanol = 9/1) to give compound I-2 (46 mg).

MS: m/z = 389.5 [M+H]⁺.

¹H NMR (500 MHz, DMSO-*d*₆) δ 12.61(s, 1H), 12.10(s, 1H), 10.67(d, J = 51.5 Hz, 1H), 7.99 (s, 1H), 7.60 (d, J = 5.5 Hz, 1H), 7.47 (dd, J = 8.5, 32.0 Hz, 1H), 7.19-7.10(m, 2H), 6.90-6.87 (m, 1H), 2.29 (s, 3H).

### Example 3: Preparation of

### 4-amino-5-(5-(4-methylpiperazin-1-yl-3,3,5,5-d₄)-1H-benzo[d]imidazol-2-yl)thieno[2,3-b] pyridin-6(7H)-one (compound I-3)

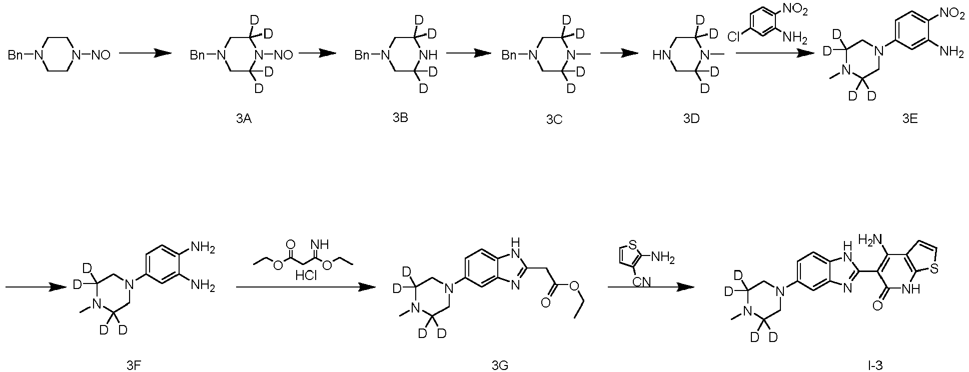

### Step 1: preparation of 1-benzyl-4-nitrosopiperazine-3,3,5,5-d₄ (compound 3A)

Heavy water (200 mL), 1-benzyl-4-nitrosopiperazine (30 g), and sodium methoxide (23.69 g) were added successively to a 500 mL reaction flask. The reaction was stirred at 80 °C under nitrogen atmosphere, and deuterated ethanol (150 mL) was then added. The reaction was stirred at 80 °C under nitrogen atmosphere for another period of time. After the reaction was completed, the reaction mixture was cooled to 0 °C and filtered under vacuum, and the filter cake was washed and dried to give compound 3A (25 g).

MS: m/z = 210.5 [M+H]⁺.

### Step 2: preparation of 1-benzylpiperazine-3,3,5,5-d₄ (compound 3B)

Compound 3A (20 g), sodium methoxide (23.23 g), deuterated ethanol (150 mL), heavy water (225 mL), and nickel-aluminum alloy (60 g) were added successively to a 1 L reaction flask, and the reaction was stirred at 30 °C. After the reaction was completed, the reaction mixture was filtered, and the filtrate was concentrated under reduced pressure. The residue was extracted with dichloromethane (100 mL × 3), and the organic phases were combined, dried, filtered, and concentrated under reduced pressure to give compound 3B (13 g).

MS: m/z = 181.5 [M+H]+.

¹H NMR (500 MHz, CDCl₃) δ 7.35-7.20 (m, 5H), 3.49 (d, J = 7.0 Hz, 2H), 2.40 (s, 4H).

### Step 3: preparation of 1-benzyl-4-methylpiperazine-3,3,5,5-d₄ (compound 3C)

Compound 3B (13 g), tetrahydrofuran (50 mL), and sodium hydride (2.88 g) were added successively to a 100 mL reaction flask at 0 °C. The reaction was slowly warmed to 25 °C and stirred for 0.5 h. Then methyl 4-methylbenzenesulfonate (6.71 g) was added to the reaction mixture at 0 °C, and the reaction was stirred at 25 °C. After the reaction was completed, the reaction mixture was quenched and concentrated under reduced pressure. The residue was separated by column chromatography (dichloromethane/methanol = 30/1) to give compound 3C (4.5 g).

MS: m/z = 195.5 [M+H]⁺.

### Step 4: preparation of 1-methylpiperazine-2,2,6,6-d₄ (compound 3D)

Compound 3C (4.5 g), 10% palladium on carbon (4 g), and absolute methanol (50 mL) were added successively to a 100 mL reaction flask, and the reaction was stirred at 25 °C under hydrogen atmosphere. After the reaction was completed, the reaction mixture was filtered, and the filtrate was concentrated under reduced pressure to give compound 3D (1.3 g).

MS: m/z =105.4 [M+H]⁺.

### Step 5: preparation of 5-(4-methylpiperazin-1-yl-3,3,5,5-d₄)-2-nitroaniline (compound 3E)

Compound 3D (1.3 g), 5-chloro-2-nitroaniline (2.15 g), potassium carbonate (6.9 g), and *N*-methylpyrrolidone (40 mL) were added successively to a 100 mL reaction flask. The reaction was stirred at 160 °C. After the reaction was completed, the reaction mixture was concentrated under reduced pressure. The residue was separated by column chromatography (dichloromethane/methanol = 9/1) to give compound 3E (1.4 g).

MS: m/z =241.2 [M+H]⁺.

### Step 6: preparation of 4-(4-methylpiperazin-1-yl-3,3,5,5-d₄)benzene-1.2-diamine (compound 3F)

Compound 3E (1.4 g), zinc powder (1.9 g), ammonium chloride (0.31 g), ethanol (30 mL), and water (5 mL) were added successively to a 100 mL reaction flask. The reaction was stirred at 80 °C. After the reaction was completed, the reaction mixture was filtered under vacuum, and the filtrate was concentrated under reduced pressure to give compound 3F (1.2 g).

MS: m/z =211.3 [M+H]⁺.

### Step 7: preparation of ethyl 2-(5-(4-methylpiperazin-1-yl-3,3,5,5-d₄)-1H-benzo[d]imidazol-2-yl)acetate (compound 3G)

Compound 3F (1.2 g), ethyl 3-ethoxy-3-iminopropionate hydrochloride (2.23 g), and ethanol (30 mL) were added successively to a 100 mL reaction flask. The reaction was stirred at 80 °C. After the reaction was completed, the reaction mixture was concentrated under reduced pressure. The residue was separated by column chromatography (dichloromethane/methanol = 30/1) to give compound 3G (0.5 g).

MS: m/z =306.9 [M+H]⁺.

### Step 8: preparation of 4-amino-5-(5-(4-methylpiperazin-l-yl-3,3,5,5-d₄)-1H-benzo[d]imidazol-2-yl)thieno[2,3-b] pyridin-6(7H)-one (compound I-3)

Compound 3G (0.5 g), 2-aminothiophene-3-carbonitrile (0.32 g), tetrahydrofuran (20 mL), and lithium diisopropylamide (1.75 g) were added successively to a 100 mL reaction flask, and the reaction was stirred at 60 °C under nitrogen atmosphere. After the reaction was completed, the reaction mixture was quenched with saturated ammonium chloride and extracted with ethyl acetate (30 mL × 3), and the organic phases were combined, dried, filtered, and concentrated under reduced pressure. The residue was separated by column chromatography (dichloromethane/methanol = 9/1) to give compound I-3 (0.1 g).

MS: m/z = 385.5 [M+H]⁺.

¹H NMR (500 MHz, DMSO-*d*₆) δ 12.62 (s, 1H), 12.10 (s, 1H), 10.67 (d, J = 47.3 Hz, 1H), 8.01 (s, 1H), 7.60 (d, J = 5.6 Hz, 1H), 7.48 (dd, J = 32.3, 8.4 Hz, 1H), 7.17 (dd, J = 24.0, 18.4 Hz, 2H), 6.90 (s, 1H), 3.13 (s, 4H), 2.33 (s, 3H).

### Example 4: Preparation of

### 4-amino-5-(5-(4-methylpiperazin-1-yl-2,2,6,6-d₄)-1H-benzo[d]imidazol-2-yl)thieno[2,3-b] pyridin-6(7H)-one (compound I-4)

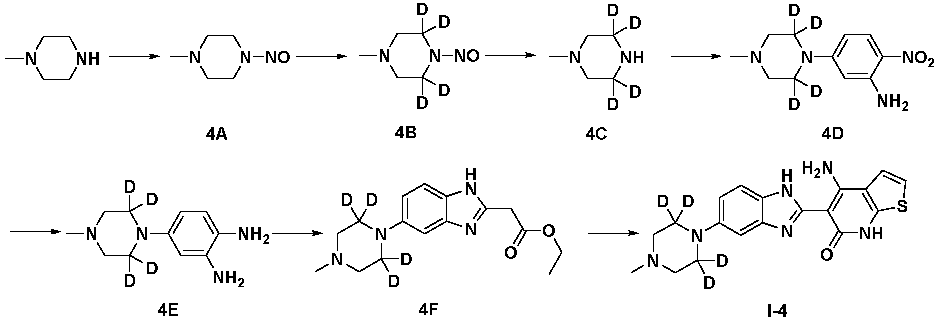

### Step 1: preparation of 1-methyl-4-nitrosopiperazine (compound 4A)

1-Methylpiperazine (10 g), water (20 mL), and concentrated hydrochloric acid (25 mL) were added successively to a 100 mL reaction flask, and a solution of sodium nitrite (8.27 g) in water (20 mL) was added to the reaction mixture at 0 °C. The reaction was stirred at 25 °C. After the reaction was completed, the reaction mixture was adjusted to alkalinity and extracted with dichloromethane (100 mL × 3), and the organic phases were combined, dried, filtered, and concentrated under reduced pressure to give compound 4A (10.8 g).

MS: m/z = 130.2 [M+H]⁺.

### Step 2: preparation of 1-methyl-4-nitrosopiperazine-3,3,5,5-d₄ (compound 4B)

Heavy water (30 mL), compound 4A (5 g), and deuterated sodium hydroxide (5.29 g) were added successively to a 100 mL reaction flask, and the reaction was stirred at 100 °C under nitrogen atmosphere. After the reaction was completed, the reaction mixture was extracted with dichloromethane (50 mL × 3), and the organic phases were combined, dried, filtered, and concentrated under reduced pressure to give compound 4B (5.3 g).

¹H NMR (500 MHz, CDCl₃) δ 2.57 (s, 2H), 2.33 (s, 2H), 2.31 (s, 3H).

### Step 3: preparation of 1-methylpiperazine-3,3,5,5-d₄ (compound 4C)

Compound 4B (5 g), sodium methoxide (15.2 g), deuterated ethanol (23 mL), heavy water (23 mL), and nickel-aluminum alloy (15 g) were added successively to a 100 mL reaction flask, and the reaction was stirred at 30 °C. After the reaction was completed, the reaction mixture was filtered, and the filtrate was concentrated under reduced pressure. The residue was extracted with dichloromethane (100 mL × 3), and the organic phases were combined, dried, filtered, and concentrated under reduced pressure to give compound 4C (4 g).

MS: m/z = 105.5 [M+H]⁺.

### Step 4: preparation of 5-(4-methylpiperazin-1-yl-2,2,6,6-d₄)-2-nitroaniline (compound 4D)

Compound 4C (3.677 g), 5-chloro-2-nitroaniline (5.413 g), potassium carbonate (10.847 g), and N-methylpyrrolidone (50 mL) were added successively to a 100 mL reaction flask, and the reaction was stirred at 160 °C. After the reaction was completed, the reaction mixture was concentrated under reduced pressure. The residue was separated by column chromatography (dichloromethane/methanol = 9/1) to give compound 4D (1.410 g).

MS: m/z = 241.2 [M+H]⁺.

### Step 5: preparation of 4-(4-methylpiperazin-1-yl-2,2,6,6-d₄)benzene-1.2-diamine (compound 4E)

4D (1.400 g), ethanol (30 mL), water (5 mL), zinc powder (2.245 g), and ammonium chloride (0.400 g) were added successively to a 100 mL reaction flask. The reaction was stirred at 80 °C. After the reaction was completed, the reaction mixture was filtered under vacuum, and the filtrate was concentrated under reduced pressure to give compound 4E (1.403 g).

MS: m/z = 211.3 [M+H]⁺.

### Step 6: preparation of ethyl 2-(5-(4-methylpiperazin-1-yl-2,2,6,6-d₄)-1H-benzo[d]imidazol-2-yl)acetate (compound 4F)

Ethyl 3-ethoxy-3-iminopropionate hydrochloride (2.230 g), compound 4E (1.400 g), and ethanol (50 mL) were added successively to a 100 mL reaction flask, and the reaction was stirred at 80 °C under nitrogen atmosphere. After the reaction was completed, the reaction mixture was concentrated under reduced pressure. The residue was separated by column chromatography (dichloromethane/methanol = 4/1) to give compound 4F (1.241 g).

MS: m/z = 307.3 [M+H]⁺.

### Step 7: preparation of 4-amino-5-(5-(4-methylpiperazin-1-yl-2,2,6,6-d₄)-1H-benzo[d]imidazol-2-yl)thieno[2,3-b] pyridin-6(7H)-one (compound I-4)

Compound 4F (1.241 g), 2-aminothiophene-3-carbonitrile (0.600 g), tetrahydrofuran (40 mL), and a 2 M solution of lithium diisopropylamide in tetrahydrofuran (20 mL) were added successively to a 100 mL reaction flask, and the reaction was stirred at 60 °C under nitrogen atmosphere. After the reaction was completed, the reaction mixture was quenched with saturated ammonium chloride and extracted with ethyl acetate, and the organic phases were combined, dried, filtered, and concentrated under reduced pressure. The residue was separated by column chromatography (dichloromethane/methanol = 9/1) to give compound I-4 (70 mg).

MS: m/z = 385.5 [M+H]⁺.

¹H NMR (500 MHz, DMSO-*d*₆) δ 12.61(s, 1H), 12.10(s, 1H), 10.69(d, J = 51.0 Hz, 1H), 7.99 (s, 1H), 7.60 (d, J = 5.5 Hz, 1H), 7.47 (dd, J = 9.0, 32.5 Hz, 1H), 7.19-7.10(m, 2H), 6.90-6.87 (m, 1H), 2.51-2.48 (m, 4H), 2.24 (s, 3H).

### Example 5: Preparation of 4-amino-5-(5-(4-methylpiperazin-1-yl)-1H-benzo[d]imidazol-2-yl-4,6-d₂)thieno[2,3-b]pyri din-6(7H)-one-2-d (compound I-11)

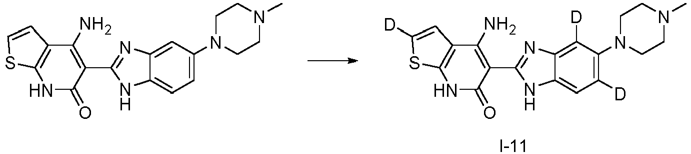

### Step 1: preparation of 4-amino-5-(5-(4-methylpiperazin-1-yl)-1H-benzo[d]imidazol-2-yl-4,6-d₂)thieno[2,3-b]pyri din-6(7H)-one-2-d (compound 1-11)

Compound 4-amino-5-(5-(4-methylpiperazin-1-yl)-1*H*-benzo[*d*]imidazol-2-yl-4,6)thieno[2,3-*b*]pyridin -6(*7H*)-one (0.3 g), heavy water (8 mL) and trifluoroacetic anhydride (0.11 mL) were added successively to a 25 mL microwave tube. After the addition was completed, the reaction mixture was microwaved to 140 °C and stirred. After the reaction was completed, the reaction mixture was adjusted to alkalinity and extracted with ethyl acetate (30 mL × 3), and the organic phases were combined, dried, filtered, and concentrated under reduced pressure. The residue was separated by column chromatography (dichloromethane/methanol = 9/1) to give compound I-11 (0.197 g).

MS: m/z = 384.5 [M+H]⁺.

¹H NMR (500 MHz, DMSO-*d*₆) δ 12.64 (s, 1H), 12.12 (s, 1H), 10.68 (d, J = 48.1 Hz, 1H), 8.04 (s, 1H), 7.62 (s, 1H), 7.49 (d, J = 32.3 Hz, 1H), 3.21-3.12 (m, 4H), 2.69 (d, J = 12.0 Hz, 4H), 2.37 (d, J = 8.3 Hz, 3H).

### Experimental Example 1: Assay for In Vitro Inhibitory Activity Against HPK1 Kinase

The kinase buffer (Enzymatic buffer 5×) was diluted to 1×, and 10 mM MgCl₂, 1 mM DTT, and 0.005% Tween 20 were added. The 100 ng/µL HPK1 (Life technology) stock solution was diluted with the kinase buffer to obtain a 1.67×, 1.67 ng/µL working solution (final concentration: 1 ng/µL), and the working solution was seeded in a 384-well plate at 6 µL/well. Different compounds dissolved in DMSO were added to the wells using a nanoliter pipettor to final concentrations of 1000 nM to 0.244 nM (4-fold serial dilution, 7 concentrations in total), and blank control wells (without enzyme) and negative control wells (with enzyme, plus vehicle DMSO) were set; the wells were set in duplicate. After the enzyme and compounds were incubated at room temperature for 1 h, a 5×, 0.5 mM ATP dilution (final concentration: 0.1 mM) in the kinase buffer was mixed with a 5×, 2.5 µM substrate (Cisbio, STK Substrate 1-biotin; final concentration: 500 nM) in equal volume, and the mixture was added to each well at 4 µL/well. The plate was sealed with a film and then incubated at room temperature for 2 h. An assay antibody solution was prepared by mixing the antibody STK Antibody-cryptate (Cisbio, 5 µL/test) and a 4×, 500 nM Streptavidin-XL665 (Cisbio; final concentration: 125 nM) in equal volume and was added to each well at 10 µL/well, and the plate was incubated at room temperature for 1 h. The signal values (excitation: 665 nm, emission: 620 nm) were measured using a PE Envision multi-functional microplate reader, and IC50 was calculated by four-parameter fitting. The results are shown in Table 1.

**Table 1**

| Example | *In vitro* inhibitory activity against enzyme HPK1 IC50 (nM) |
|---|---|
| 2 | 9.7 |
| 3 | 7.5 |
| 4 | 9.2 |
| 5 | 8.8 |

### Experimental Example 2: Assay for Jurkat Cell p-SLP76 Phosphorylation Inhibitory Activity

The Jurkat cells in a good growth state were added to a centrifuge tube, centrifuged, and resuspended. The cell density was adjusted to 6.25 × 10⁶ cells/mL, and the cells were seeded in a 384-well small-volume white plate at 8 µL/well. Compounds were added in duplicate using a nanoliter pipettor to final concentrations of 2500 nM to 10.29 nM and a control was set. After 1 h of cell culture, the stimulator CD3CD28 (4 µL; manufacturer: Stemcell) was added and the cells were incubated at 37 °C for 30 min. 3 µL of lysis buffer (manufacturer: BioAuxilium) was added to each well, and the plate was shaken at room temperature for 30 min. After the lysate was well mixed, 5 µL of pre-mixed antibody (manufacturer: BioAuxilium) in assay buffer was added and the plate was incubated overnight at room temperature. The signal values were measured on a PerkinElmer Envision multi-functional microplate reader (excitation: 320 nm, emission: 615 nm/665 nm), and IC50 was calculated by four-parameter fitting. The results are shown in Table 2.

**Table 2**

| Example | Jurkat cell p-SLP76 phosphorylation inhibition IC₅₀ (nM) |
|---|---|
| 4 | 83 |
| 5 | 53 |

### Experimental Example 3: In Vitro Liver Microsomal Stability

Preparation of liver microsome incubated samples: PBS buffer (pH 7.4), liver microsome solutions (0.5 mg/mL; HLM and MLM, respectively), the test compounds and NADPH + MgCl₂ solution were mixed and incubated at 37 °C at 300 rpm for 1 h. Preparation of zero-hour samples: PBS buffer (pH 7.4), liver microsome solutions (0.5 mg/mL; HLM and MLM, respectively) and the test compounds were mixed. An acetonitrile solution containing internal standard was added to the samples, and supernatants were prepared by protein precipitation, diluted and then analyzed by LC/MS/MS. The results are shown in Table 3.

**Table 3**

| Example | HLM (0.5 mg/mL) Remaining percentage (T = 60 min) | MLM (0.5 mg/mL) Remaining percentage (T = 60 min) |
|---|---|---|
| 2 | 89.66% | 45.97% |
| 3 | 90.85% | 45.47% |
| 4 | 88.50% | 42.58% |

## Claims

1. A compound of formula (I) or a pharmaceutically acceptable salt thereof, 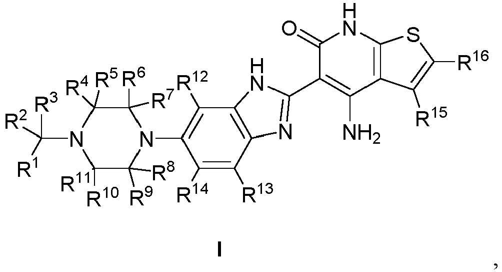
wherein R¹, R², R³, R⁴, R⁵, R⁶, R⁷, R⁸, R⁹, R¹⁰, R¹¹, R¹², R¹³, R¹⁴, R¹⁵ and R¹⁶ are each independently selected from the group consisting of hydrogen and deuterium,
provided that at least one of R¹, R², R³, R⁴, R⁵, R⁶, R⁷, R⁸, R⁹, R¹⁰, R¹¹, R¹², R¹³, R¹⁴, R¹⁵ and R¹⁶ is selected from deuterium.

2. The compound or the pharmaceutically acceptable salt thereof according to claim 1, wherein at least two, three, four, five, six, seven, eight, nine, ten, eleven, twelve, thirteen, fourteen, fifteen or sixteen of the R¹, R², R³, R⁴, R⁵, R⁶, R⁷, R⁸, R⁹, R¹⁰, R¹¹, R¹², R¹³, R¹⁴, R¹⁵ and R¹⁶ are selected from deuterium.

3. The compound or the pharmaceutically acceptable salt thereof according to claim 1, wherein any one, two, three, four, five, six, seven, eight, nine, ten, eleven, twelve, thirteen, fourteen, fifteen or sixteen of the R¹, R², R³, R⁴, R⁵, R⁶, R⁷, R⁸, R⁹, R¹⁰, R¹¹, R¹², R¹³, R¹⁴, R¹⁵ and R¹⁶ are selected from deuterium.

4. The compound or the pharmaceutically acceptable salt thereof according to any one of claims 1-3, wherein R¹, R² and R³ are selected from deuterium, and R⁴, R⁵, R⁶, R⁷, R⁸, R⁹, R¹⁰, R¹¹, R¹², R¹³, R¹⁴, R¹⁵ and R¹⁶ are each independently selected from the group consisting of hydrogen and deuterium.

5. The compound or the pharmaceutically acceptable salt thereof according to any one of claims 1-3, wherein R⁴, R⁵, R¹⁰ and R¹¹ are selected from deuterium, and R¹, R², R³, R⁶, R⁷, R⁸, R⁹, R¹², R¹³, R¹⁴, R¹⁵ and R¹⁶ are each independently selected from the group consisting of hydrogen and deuterium.

6. The compound or the pharmaceutically acceptable salt thereof according to any one of claims 1-3, wherein R⁶, R⁷, R⁸ and R⁹ are selected from deuterium, and R¹, R², R³, R⁴, R⁵, R¹⁰, R¹¹, R¹², R¹³, R¹⁴, R¹⁵ and R¹⁶ are each independently selected from the group consisting of hydrogen and deuterium.

7. The compound or the pharmaceutically acceptable salt thereof according to any one of claims 1-3, wherein R¹, R², R³, R⁴, R⁵, R¹⁰ and R¹¹ are selected from deuterium, and R⁶, R⁷, R⁸, R⁹, R¹², R¹³, R¹⁴, R¹⁵ and R¹⁶ are each independently selected from the group consisting of hydrogen and deuterium.

8. The compound or the pharmaceutically acceptable salt thereof according to any one of claims 1-3, wherein R¹, R², R³, R⁶, R⁷, R⁸ and R⁹ are selected from deuterium, and R⁴, R⁵, R¹⁰, R¹¹, R¹², R¹³, R¹⁴, R¹⁵ and R¹⁶ are each independently selected from the group consisting of hydrogen and deuterium.

9. The compound or the pharmaceutically acceptable salt thereof according to any one of claims 1-3, wherein the R⁴, R⁵, R⁶, R⁷, R⁸, R⁹, R¹⁰ and R¹¹ are selected from deuterium, and R¹, R², R³, R¹², R¹³, R¹⁴, R¹⁵ and R¹⁶ are each independently selected from the group consisting of hydrogen and deuterium.

10. The compound or the pharmaceutically acceptable salt thereof according to any one of claims 1-3, wherein R¹, R², R³, R⁴, R⁵, R⁶, R⁷, R⁸, R⁹, R¹⁰ and R¹¹ are selected from deuterium, and R¹², R¹³, R¹⁴, R¹⁵ and R¹⁶ are each independently selected from the group consisting of hydrogen and deuterium.

11. The compound or the pharmaceutically acceptable salt thereof according to any one of claims 1-3, wherein R¹² and R¹⁴ are selected from deuterium, and R¹, R², R³, R⁴, R⁵, R⁶, R⁷, R⁸, R⁹, R¹⁰, R¹¹, R¹³, R¹⁵ and R¹⁶ are each independently selected from the group consisting of hydrogen and deuterium.

12. The compound or the pharmaceutically acceptable salt thereof according to any one of claims 1-3, wherein the R¹³ is selected from deuterium, and R¹, R², R³, R⁴, R⁵, R⁶, R⁷, R⁸, R⁹, R¹⁰, R¹¹, R¹², R¹⁴, R¹⁵ and R¹⁶ are each independently selected from the group consisting of hydrogen and deuterium.

13. The compound or the pharmaceutically acceptable salt thereof according to any one of claims 1-3, wherein the R¹², R¹³ and R¹⁴ are selected from deuterium, and R¹, R², R³, R⁴, R⁵, R⁶, R⁷, R⁸, R⁹, R¹⁰, R¹¹, R¹⁵ and R¹⁶ are each independently selected from the group consisting of hydrogen and deuterium.

14. The compound or the pharmaceutically acceptable salt thereof according to any one of claims 1-3, wherein the R¹, R², R³, R¹², R¹³ and R¹⁴ are selected from deuterium, and R⁴, R⁵, R⁶, R⁷, R⁸, R⁹, R¹⁰, R¹¹, R¹⁵ and R¹⁶ are each independently selected from the group consisting of hydrogen and deuterium.

15. The compound or the pharmaceutically acceptable salt thereof according to any one of claims 1-3, wherein the R¹⁵ is selected from deuterium, and R¹, R², R³, R⁴, R⁵, R⁶, R⁷, R⁸, R⁹, R¹⁰, R¹¹, R¹², R¹³, R¹⁴ and R¹⁶ are each independently selected from the group consisting of hydrogen and deuterium.

16. The compound or the pharmaceutically acceptable salt thereof according to any one of claims 1-3, wherein the R¹⁶ is selected from deuterium, and R¹, R², R³, R⁴, R⁵, R⁶, R⁷, R⁸, R⁹, R¹⁰, R¹¹, R¹², R¹³, R¹⁴ and R¹⁵ are each independently selected from the group consisting of hydrogen and deuterium.

17. The compound or the pharmaceutically acceptable salt thereof according to any one of claims 1-3, wherein the R¹⁵ and R¹⁶ are selected from deuterium, and R¹, R², R³, R⁴, R⁵, R⁶, R⁷, R⁸, R⁹, R¹⁰, R¹¹, R¹², R¹³ and R¹⁴ are each independently selected from the group consisting of hydrogen and deuterium.

18. The compound or the pharmaceutically acceptable salt thereof according to any one of claims 1-3, wherein the R¹, R², R³, R¹⁵ and R¹⁶ are selected from deuterium, and R⁴, R⁵, R⁶, R⁷, R⁸, R⁹, R¹⁰, R¹¹, R¹², R¹³ and R¹⁴ are each independently selected from the group consisting of hydrogen and deuterium.

19. The compound or the pharmaceutically acceptable salt thereof according to any one of claims 1-18, being selected from the group consisting of the following compounds or pharmaceutically acceptable salts thereof: 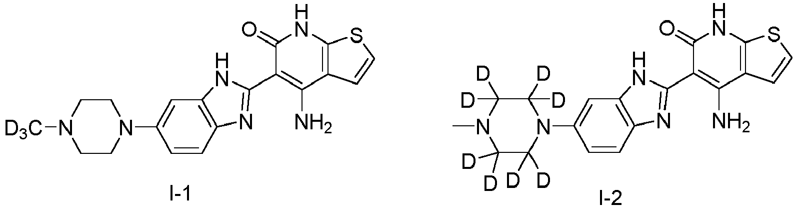 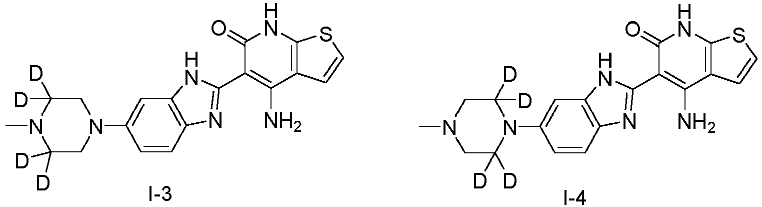 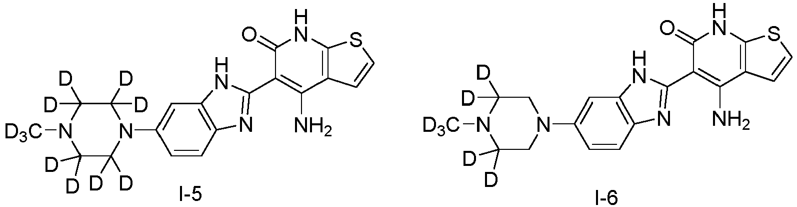 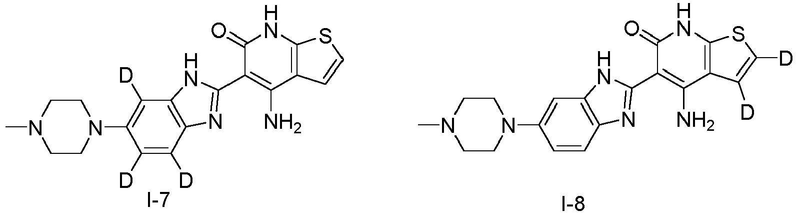 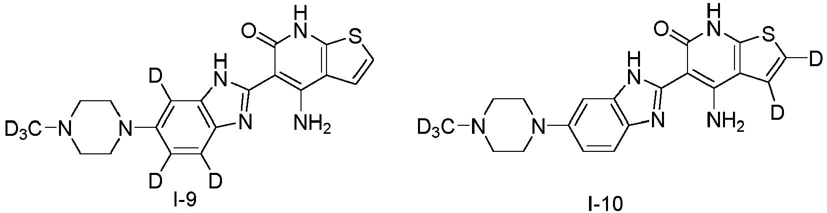 and

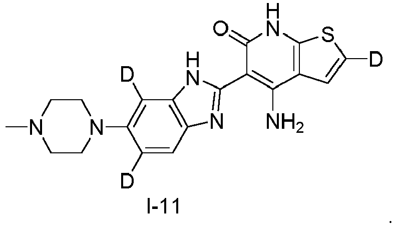

20. A pharmaceutical composition comprising the compound or the pharmaceutically acceptable salt thereof according to any one of claims 1-19 and optionally further comprising a pharmaceutically acceptable excipient.

21. The pharmaceutical composition according to claim 20 further comprising a second active agent selected from an anti-cancer agent.

22. Use of the compound or the pharmaceutically acceptable salt thereof according to any one of claims 1-19 or the pharmaceutical composition according to claim 20 for preparing a medicament for treating a disease that benefits from inhibiting HPK1 kinase activity, wherein the medicament optionally further comprises a second active agent for treating the disease, and the second active agent is selected from an anti-cancer agent.

23. The use according to claim 22, wherein the disease that benefits from inhibiting HPK1 kinase activity is selected from the group consisting of tumors and cancer.
